# EUROPÄISCHE PATENTANMELDUNG

(11) **EP 2 520 254 A1**
(43) Veröffentlichungstag der Anmeldung: **07.11.2012**
(21) Anmeldenummer: 11164736.8
(22) Anmeldetag: 04.05.2011
(51) Int. Cl.: A61F 2/46, A61F 2/00, A61F 2/30

(54) **Instrument zum Erfassen eines Angriffmittels an einem Implantat**

(71) Anmelder: Jossi Holding AG, 8546 Islikon (CH)
(72) Erfinder: Meyenhofer, Andreas, 8255, Schlattingen (CH); Gugler, Christian, 8500, Frauenfeld (CH)
(74) Vertreter: Hepp Wenger Ryffel AG

(57) **Zusammenfassung**

Das Instrument verfügt über eine wenigstens zwei Klemmbacken (18, 18') aufweisende Spannzange (19) zum Erfassen eines Angriffmittels (4) an einem Implantatkörper (1). Die Spannzange ist in einem Hülsenabschnitt (20) aufgenommen, dessen freies Ende (21) einen an der Oberfläche des Implantats abstützbaren Stützabschnitt (22) aufweist. Als Betätigungsmittel zum Betätigen der Spannzange dient ein an der Spannzange angreifendes Zugelement (23). Die Aussenseiten der Klemmbacken wirken dabei derart mit der Innenseite des Hülsenabschnitts zusammen, dass die Spannzange durch Zug am Zugelement schliessbar und dabei relativ zum freien Ende des Hülsenabschnitts verschiebbar ist.

## Beschreibung

Die Erfindung betrifft ein Instrument zum Erfassen eines Angriffmittels an einem Implantat gemäss dem Oberbegriff von Anspruch 1. Derartige Instrumente werden beispielsweise zum Freilegen einer Öffnung an einer Hüftgelenkpfanne eingesetzt. Die Instrumente können aber auch nur dazu dienen, das Implantat selber zu handhaben.

Durch die US 5,370,702 ist eine Hüftgelenkschale bekannt geworden, an der optional so genannte Schraubentrichterverschlüsse ausgebrochen werden können. Die einstückig mit der Pfanne ausgebildeten Verschlussabschnitte sind mit nach innen ragenden Zapfen versehen, die als Angriffmittel für ein Werkzeug dienen. Mit Hilfe dieses Werkzeugs können Zug- oder Biegekräfte auf den Verschlussabschnitt ausgeübt werden, bis dieser im Bereich einer umlaufenden Schwächungszone von der Pfanne abgelöst werden kann. Ein dazu geeignetes Werkzeug ist allerdings nicht beschrieben.

Die US 4,263,903 beschreibt eine Vorrichtung zum Halten von medizinischen Heftklammern. Diese Vorrichtung weist eine Spannzange auf, mit welcher die U-förmigen Heftklammern erfasst werden können. Die Spannzange ist in Öffnungsstellung vorgespannt und kann durch lineare Verschiebung im Innern einer Hülse geschlossen werden. Als Zugelement zum Verschieben der Spannzange dient eine Schraubenmutter, bei deren Drehung ein Schraubenbolzen verschoben wird. Dieses Instrument eignet sich nicht zum Erfassen eines Implantats und insbesondere auch nicht zum Freilegen der oben beschriebenen Schraubentrichterverschlüsse.

Die DE 10 2008 022 329 beschreibt ein Handhabungssystem für eine Hüftgelenkpfanne. Das Instrument verfügt ebenfalls über eine Spannzange, die einen zentralen Bolzen an der Hüftgelenkpfanne ergreifen kann. Das Instrument verfügt ferner über einen Hülsenabschnitt, dessen Stirnseite an einer korrespondierenden Auflagefläche abstützbar ist. Damit wird eine Verspannung zwischen Spannzange und Implantat erreicht. Die Spannzange wird indirekt über eine Spannhülse betätigt, was einen relativ komplizierten Aufbau erfordert. Ausserdem eignet sich dieses Instrument nicht zum Ausbrechen von Schraubentrichterverschlüssen.

Es ist daher eine Aufgabe der Erfindung, ein Instrument der eingangs genannten Art zu schaffen, das einfach aufgebaut und zuverlässig in der Handhabung ist. Das Instrument soll auch ein Freilegen von relativ kleinen Öffnungen ermöglichen, und es soll mit geringem Kraftaufwand betätigbar sein. Diese Aufgabe wird erfindungsgemäss mit einem Instrument gelöst, das die Merkmale im Anspruch 1 aufweist. Das direkt an der Spannzange angreifende Zugelement ermöglicht eine einfache und Platz sparende Konstruktion. Die Aussenseiten der Klemmbacken wirken dabei derart mit der Innenseite des Hülsenabschnitts zusammen, dass die Spannzange durch Zug am Zugelement schliessbar und dabei relativ zum freien Ende des Hülsenabschnitts verschiebbar ist. Das freie Ende weist dabei einen Stützabschnitt auf, der an der Oberfläche des Implantats abstützbar ist. Das Implantat dient dabei als Widerlager zur Aufnahme der mit der Spannzange ausgeübten Zugkräfte. Dadurch werden keine schädlichen Kräfte auf eine bereits implantierte Schale ausgeübt und auch das Instrument selbst wird nicht unnötig beansprucht.

Der Stützabschnitt ist vorzugsweise kegelstumpfförmig ausgebildet, womit ein sicherer Sitz gewährleistet ist. Die Kegelstumpfform entspricht dabei vorzugsweise der Konfiguration des Schraubentrichters an einem Schraubentrichterverschluss.

Die vorstehend beschriebene Wechselwirkung zwischen der Aussenseite der Klemmbacken und der Innenseite des Hülsenabschnitts kann vorteilhaft dadurch erreicht werden, dass der Hülsenabschnitt auf der Innenseite eine Verjüngung aufweist, die mit Aussenseiten der Klemmbacken zusammenwirkt. Zusätzlich kann auf der Innenseite des Hülsenabschnitts wenigstens ein vorzugsweise zylindrischer Führungsabschnitt zum Führen der Spannzange angeordnet sein. Auf diese Weise dient der Hülsenabschnitt der Geradeführung und der Spannung der Spannzange.

Der Hülsenabschnitt kann am Ende eines Hohlschaftes angeordnet und vorzugsweise einstückig mit diesem verbunden sein, wobei sich das Zugelement durch den Hohlschaft erstreckt. Der Hohlschaft kann dabei auch unmittelbar als Griff ausgebildet sein, wie dies beispielsweise aus der US 4,263,903 ersichtlich ist. Der Hohlschaft kann aber auch nur der Führung des Zugmittels dienen.

Der Hülsenabschnitt kann gegenüber dem Hohlschaft mittels einer Krümmung abgewinkelt sein. Diese Abwinklung erleichtert an einer Hüftgelenkpfanne den Zugriff auf Schraubentrichterverschlüsse, die nahe am Äquator angeordnet sind. Dies gilt insbesondere für Hüftschalen, die bereits implantiert sind und bei denen der Zugang durch den Eingriff nicht unnötig vergrössert werden sollte. Für die heute üblichen, minimalinvasiven Operationstechniken ist das Instrument daher sehr geeignet. Die Krümmung kann selbstverständlich den jeweiligen Gegebenheiten am individuellen Implantat und/oder der Operationstechnik angepasst werden. Die Abwinklung kann beispielsweise etwa 60° betragen.

Weitere Vorteile können erreicht werden, wenn im Innenbereich der Krümmung ein Gleitschuh mit einer gekrümmten Gleitoberfläche zur Umlenkung des Zugelements angeordnet ist. Je nach Materialpaarung kann mit Hilfe des Gleitschuhs die Reibung stark reduziert werden. Dies ist besonders wichtig, weil Schmiermittel an chirurgischen Instrumenten ersichtlicherweise nicht zulässig sind. Eine Reduktion der Reibung lässt sich zusätzlich oder alternativ auch durch eine besondere Konfiguration der Krümmung bewirken, indem die Krümmung zumindest im Bereich der gleitenden Reibung am Zugmittel nicht als Kreisausschnitt verläuft, sondern vom Scheitelpunkt der Krümmung gegen die geraden Abschnitte abgeflacht ist. Die Krümmung könnte beispielsweise als Doppelevolvente, Doppelklothoide oder in einer anderen geeigneten Kurvenform ausgebildet sein.

Da derartige Instrumente in geeigneten Vorrichtungen gereinigt und sterilisiert werden müssen, ist es besonders zweckmässig, wenn im Aussenbereich der Krümmung eine sich vorzugsweise über die gesamte Krümmung erstreckende Öffnung angeordnet ist. Über diese Öffnung ist gewährleistet, dass Reinigungsflüssigkeit bzw. Sterilisationsmittel sämtliche Innenwandbereiche bestreichen kann.

Eine besonders einfache Bauweise ergibt sich, wenn die Klemmbacken der Spannzange an einem Führungsabschnitt einstückig miteinander verbunden sind. Die Klemmbacken bewegen sich relativ zueinander ausschliesslich durch Eigenfederung des Materials, so dass keine zusätzlichen mechanischen Teile erforderlich sind. Die Spannzange lässt sich dadurch auch leichter reinigen und sterilisieren. Auf der Aussenseite können die Klemmbacken im Abstand zum Führungsabschnitt einen Klemmrampenabschnitt aufweisen, der mit der Innenseite des Hülsenabschnitts zusammenwirkt. Die Distanz zwischen dem Führungsabschnitt und dem Klemmrampenabschnitt definiert somit einen Hebelarm, um den sich die Klemmbacken aufeinander zu bewegen.

Für ein besonders sicheres Ansetzen der Klemmbacken können diese auf der Innenseite eine Hinterschneidung zum Erfassen eines Vorsprungs am Angriffmittel des Implantats aufweisen. Bei entsprechender Konfiguration kann das Angriffmittel nicht mehr aus den geschlossenen Klemmbacken entfernt werden. Alternativ könnten die Klemmbacken natürlich auf der Innenseite auch Rippen oder Dorne zum besseren Erfassen des Angriffmittels aufweisen.

Das Zugelement ist vorteilhaft als Zugstange ausgebildet, die wenigstens abschnittweise im Hohlschaft geführt ist. Mit der Zugstange können am Zugelement Kräfte in beide Richtungen problemlos übertragen werden. Alternativ könnte das Zugelement selbstverständlich auch als Seilzug ausgebildet sein. Die Zugstange oder aber auch der Seilzug können einen Einspannabschnitt zum Einspannen in eine Zugvorrichtung aufweisen. Auf diese Weise kann die eigentliche Zugvorrichtung vom Zugelement getrennt werden bzw. das Zugelement kann mit verschiedenen Zugvorrichtungen gekoppelt werden.

Insbesondere bei einem gekrümmten Verlauf des Zugelements ist es zweckmässig, wenn dieses wenigstens abschnittweise flexibel, vorzugsweise in der Form einer Blattfeder ausgebildet ist. Eine Blattfeder lässt sich um eine parallel zur Breitseite verlaufende Achse relativ leicht krümmen, und mit ihr können gleichermassen Schub- und Zugkräfte übertragen werden.

Die Herstellung und Reinigung des Instruments kann weiter dadurch vereinfacht werden, dass die Spannzange einstückig mit dem Zugelement ausgebildet ist. Das Instrument besteht dadurch abgesehen von der eigentlichen Zugvorrichtung lediglich aus zwei Bestandteilen, nämlich aus dem Hohlschaft mit einstückig ausgebildetem Hülsenabschnitt und aus der Spannzange mit einstückig ausgebildetem Zugelement.

Ein hoher Grad an Betriebssicherheit insbesondere bei der Handhabung des Instruments am Patienten kann dadurch erreicht werden, dass der Hülsenabschnitt und das Zugelement direkt oder indirekt sowie lösbar mit einer Zugvorrichtung verbunden sind, an der eine aufgebaute Zugkraft derart aufrechterhaltbar ist, dass sich die geschlossene Spannzange erst nach dem Lösen einer Arretierung öffnet. Dadurch wird verhindert, dass sich die Spannzange nach dem Herauslösen eines Verschlussabschnitts unbeabsichtigt öffnet und der Abschnitt so in den Operationsbereich gelangen kann. Erst nach dem bewussten und gewollten Lösen der Arretierung kann der ausgebrochene Verschlussabschnitt aus der Spannzange entfernt und entsorgt werden. Diese Funktion ist selbstverständlich auch dann nützlich, wenn das Instrument nicht in vivo eingesetzt wird.

Es hat sich als besonders vorteilhaft erwiesen, wenn die Zugvorrichtung eine vorzugsweise handbetätigte Blindnietzange ist, bzw. wenn sie deren Funktionen ausüben kann. Bekanntlich sind diese Zangen in der Lage, den Dorn eines Blindniets zu erfassen und in die Zange hineinzuziehen, wobei eine definierte Zugkraft aufbringbar ist. Ausserdem verfügen derartige Zangen auch über eine Arretierung zum Lösen des abgebrochenen Nietdorns und zum Wiederherstellen der Ausgangslage zum Erfassen eines neuen Dorns. Diese Eigenschaften lassen sich ideal dazu verwenden, um die Zugvorrichtung am erfindungsgemässen Instrument zu betätigen, wobei selbstverständlich Anpassungen für den Einsatz im medizinischen Bereich erforderlich sind. Die Erfindung betrifft somit auch die Verwendung einer vorzugsweise handbetätigten Blindnietzange als Zugvorrichtung zum Betätigen eines Instruments zum Erfassen eines Angriffmittels an einem Implantat. Andere Kraftgeneratoren als Zugvorrichtung sind jedoch denkbar. Diese können von Hand, elektromotorisch oder pneumatisch betätigbar sein.

Schliesslich betrifft die Erfindung auch eine Anordnung bestehend aus einem Implantat sowie aus einem Instrument mit den Merkmalen im Anspruch 17.

Ein weiteres vorteilhaftes Instrument im Rahmen der vorliegenden Erfindung weist die Merkmale im Anspruch 18 auf. Das Instrument verfügt über einen länglichen Griff sowie über Eingriffmittel. Die Eingriffmittel sind wieder lösbar mit dem Verschlussabschnitt verbindbar.

Über den länglichen Griff kann eine Kraft ausgeübt werden, um den Verschlussabschnitt aus der Wand des Implantats zu entfernen. Die Eingriffmittel dienen dabei einerseits zum Übertragen der Kraft vom Griff auf den Verschlussabschnitt, und anderseits kann der Verschlussabschnitt damit sicher und einfach aus der Implantatwand entfernt werden.

Bevorzugt sind die Eingriffmittel derart ausgebildet, dass diese mit einem auf dem Verschlussabschnitt angeordneten Angriffmittel verbindbar sind. Dadurch kann der Verschlussabschnitt unverlierbar mit dem Instrument verbunden werden, was auch eine Entfernung eines Verschlussabschnitts bei bereits eingesetztem Implantat ermöglicht.

Ein erfindungsgemässer Verschlussabschnitt kann auch durch Beaufschlagen mit Druck- oder Zugkräften entfernt werden. Dies kann beispielsweise durch Drücken oder Schlagen sowie durch Ziehen erfolgen. Alternativ kann ein erfindungsgemässer Verschlussabschnitt auch durch Biege- oder Drehkräfte entfernt werden.

Vorteile und Einzelmerkmale der Erfindung werden nachstehen genauer beschrieben und in den Zeichnungen dargestellt. Es zeigen:
- Figur 1:: einen schematischen Querschnitt durch ein Implantat,
- Figur 2:: einen Querschnitt durch einen Verschlussabschnitt mit Sollbruchlinie in stark vergrösserter Darstellung,
- Figuren 3a - 3e:: fünf verschiedene schematische Querschnitte durch entfernbare Verschlussabschnitte mit Sollbruchlinien,
- Figur 4:: eine perspektivische und teilweise aufgeschnittene Darstellung einer Hüftgelenkpfanne mit mehreren Verschlussabschnitten,
- Figur 5:: die Hüftgelenkpfanne gemäss Figur 4 mit einem abgelösten Verschlussabschnitt,
- Figur 6:: die Hüftgelenkpfanne gemäss Figur 5 mit einer in die freigelegte Öffnung eingesetzten Schraube,
- Figur 7:: ein Teilquerschnitt durch ein erfindungsgemässes Instrument beim Erfassen eines Verschlussabschnitts,
- Figur 8:: ein Teilquerschnitt durch einen Hohlschaft mit abgewinkeltem Hülsenabschnitt,
- Figur 9:: eine Draufsicht auf den Hohlschaft gemäss Figur 8 im Bereich der Krümmung,
- Figur 10:: einen Querschnitt durch einen Hülsenabschnitt mit Spannzange vor dem Ansetzen auf das Implantat,
- Figur 11:: den Hülsenabschnitt gemäss Figur 10 im angesetzten Zustand,
- Figur 12:: eine Seitenansicht auf eine Spannzange mit Zugelement,
- Figur 13:: die Spannzange mit Zugelement gemäss Figur 12 um 90° gedreht,
- Figur 14:: eine Ansicht auf die Stirnseite der Spannzange gemäss Figur 12 in vergrössertem Massstab,
- Figur 15: einen Querschnitt durch die Spannzange gemäss Figur 12 in vergrössertem Massstab,
- Figur 16: die Gesamtdarstellung einer Anordnung bestehend aus Implantatkörper, Instrument und Zugvorrichtung,
- Figur 17: einen Querschnitt durch die Zugvorrichtung gemäss Figur 16, und
- Figur 18: einen Querschnitt durch ein abgewandeltes Ausführungsbeispiel eines Instruments.

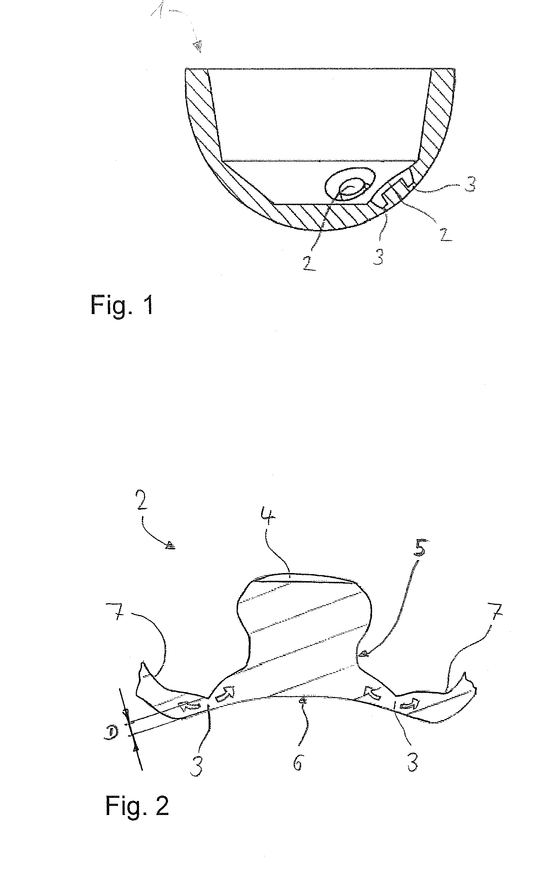

Figur 1 stellt einen schematischen Querschnitt eines Implantats 1 dar, bei welchem mit Hilfe einer Materialumformung durch Sollbruchlinien 3 abgegrenzte Verschlussabschnitte 2 angebracht sind. Exemplarisch ist das Implantat 1 als Hüftgelenkpfanne ausgebildet. Alternativ kann es sich beim Implantat jedoch auch um ein anderes Implantat handeln, wie beispielsweise eine Osteosyntheseplatte. Beispielhaft weist das Implantat 1 zwei Verschlussabschnitte 2 auf. Ein Implantat 1 kann jedoch auch nur einen Verschlussabschnitt 2 oder mehr als zwei Verschlussabschnitte 2 aufweisen. Die Verschlussabschnitte 2 können dabei in beliebiger Anordnung auf der Oberfläche des Implantats 1 angeordnet werden.

Figur 2 zeigt einen schematischen Schnitt durch einen Verschlussabschnitt eines Implantats, bei welchem mit einem Umformverfahren ein durch eine Sollbruchlinie 3 abgegrenzter Verschlussabschnitt 2 angebracht wurde. Die Sollbruchlinie 3 weist gegenüber dem umgebenden Wandabschnitt 7 eine geringere Materialstärke D auf. Typischerweise ist die Materialstärke D dabei kleiner als 1 mm. Der durch das Umformverfahren erfolgte Materialfluss, exemplarisch durch Pfeile dargestellt, wird dabei bevorzugt gezielt in den Verschlussabschnitt 2 geleitet. Alternativ kann der Materialfluss aber auch gezielt in den umgebenden Wandabschnitt geleitet werden. Der Verschlussabschnitt 2 weist ein Angriffmittel 4 auf, welches in diesem Beispiel als Materialvorsprung mit Hinterschnitt 5 ausgebildet ist. Das Angriffmittel 4 kann alternativ auch andere Formen aufweisen wie z. B. die Form eines Zylinders oder eines Kegelstumpfs. Der Verschlussabschnitt 2 weist dabei zusätzlich eine konkave Wölbung 6 auf. Die Wölbung 6 kann alternativ auch konvex oder gewellt ausgestaltet sein. Der Verschlussabschnitt 2 kann alternativ auch flach ausgestaltet sein.

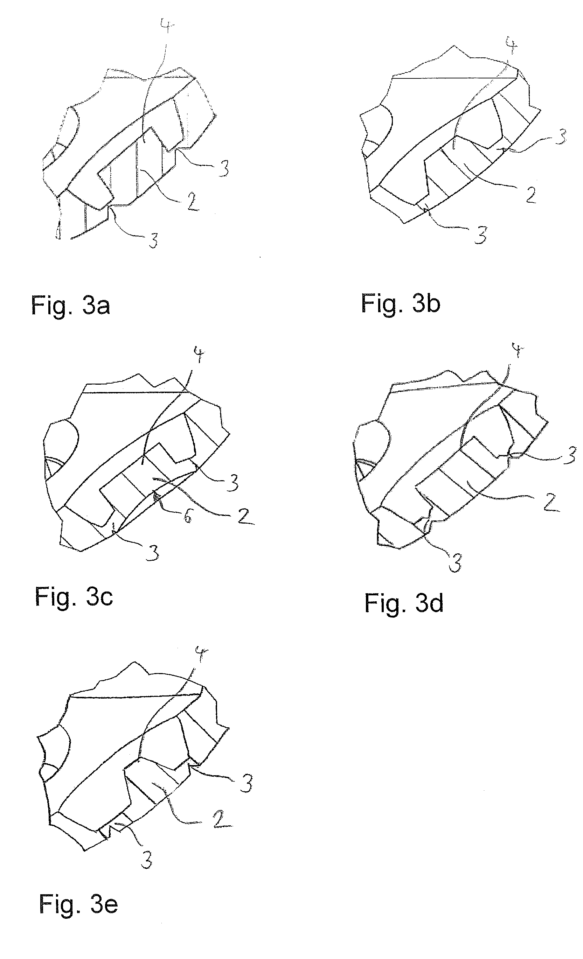

Die Figuren 3a bis 3e zeigen verschiedene Ausführungsformen von Verschlussabschnitten 2 eines Implantats. Die Ausführungsform der Figur 3a weist dabei ein Angriffmittel 4 auf, welches zylindrisch oder prismatisch ausgebildet ist. Figur 3b zeigt eine Ausführungsform mit kegelstumpfförmigem Angriffmittel 4. Die Sollbruchlinie 3, die den Verschlussabschnitt 2 abgrenzt, weist bei dieser Ausführungsform eine grössere Materialstärke auf als die Ausführungsform der Figur 3a. Eine weitere Variante zeigt

Figur 3c. Bei dieser Variante weist die Aussenseite des Verschlussabschnitts 4 eine konvexe Wölbung 6 auf. Der in Figur 3a gezeigte Verschlussabschnitt 2 kann dabei einen Zwischenschritt bei der Herstellung des Verschlussabschnitts 2 der Figur 3c sein. Figur 3d zeigt eine weitere Ausführungsform eines Verschlussabschnitts 2, bei dem die Materialstärke der Sollbruchlinie 3 auf beiden Seiten der Wand des Implantats reduziert wurde. Figur 3e zeigt eine weitere Ausführungsform eines Verschlussabschnitts 3. Bei dieser Ausführungsform ist das Angriffmittel 4 nicht zentralsymmetrisch auf dem Verschlussabschnitt 2 angeordnet, sondern exzentrisch. Die einzelnen Merkmale der beschriebenen Ausführungsformen des Verschlussabschnitts 2 können je nach Bedarf miteinander kombiniert werden. Auch können verschiedene Ausführungsformen eines Verschlussabschnitts 2 auf demselben Implantat vorhanden sein. Alternativ zu einer Schraube können auch andere Elemente, wie z.B. Nägel oder Stifte eingesetzt werden oder die Öffnung kann für besondere chirurgische Eingriffe benutzt werden.

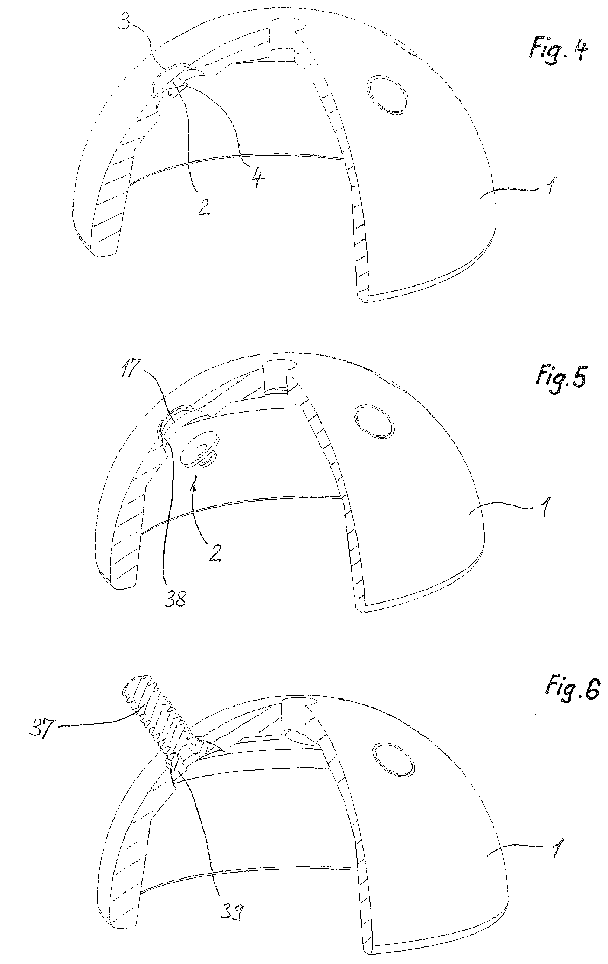

Die Figuren 4 bis 6 zeigen ein alternatives Ausführungsbeispiel eines Implantatkörpers 1 in der Form einer Hüftgelenkpfanne. Neben der zentralen Öffnung im Polbereich sind in der Schale einstückig mehrere Verschlussabschnitte 2 angeformt, welche etwa analog zur Darstellung gemäss Figur 2 durch eine Sollbruchstelle 3 abgegrenzt sind und welche je ein nach innen ragendes Angriffmittel 4 aufweisen. Die Verschlussabschnitte können wahlweise herausgebrochen werden, wobei ein Schraubentrichter 38 freigelegt wird und eine Öffnung 17 entsteht (Figur 5). In diese Öffnung kann eine Schraube 37 eingesetzt werden, deren Schraubenkopf 39 komplementär zum Schraubentrichter 38 ausgebildet ist (Figur 6). Alternativ zu einer Schraube können auch andere Elemente, wie z.B. Nägel oder Stifte eingesetzt werden oder die Öffnung kann für besondere chirurgische Eingriffe benutzt werden. Die Aussenseite der Hüftgelenkpfanne kann mit unterschiedlichen Strukturen zur Verbesserung der Haftung versehen sein.

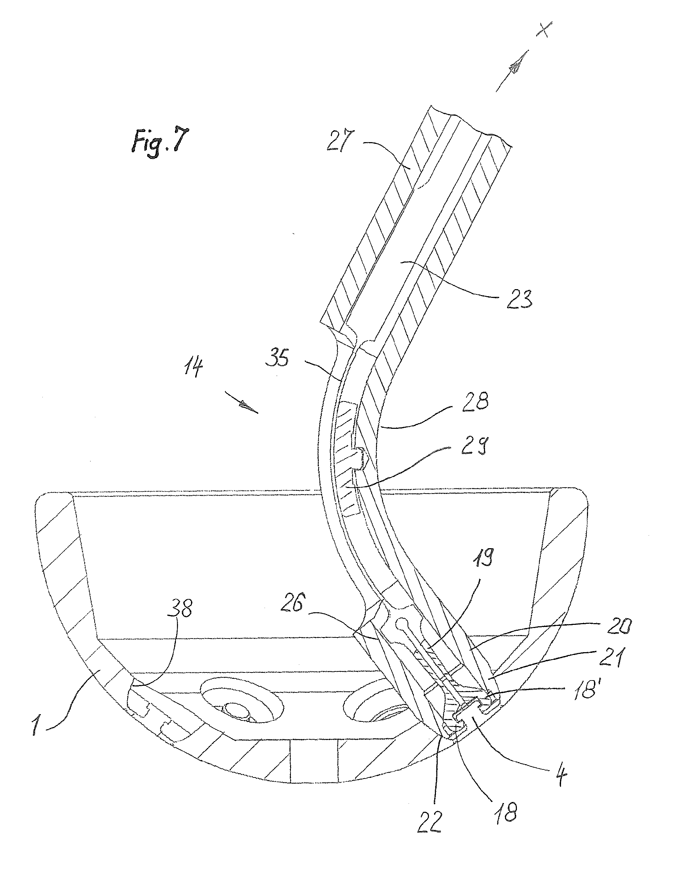

Figur 7 zeigt ein erfindungsgemässes Instrument 14, das zum Freilegen einer Öffnung am Implantatkörper 1 mit einer Spannzange 19 an einem Angriffmittel 4 angreift. Diese Spannzange verfügt über zwei Klemmbacken 18, 18', und sie ist in einem Hülsenabschnitt 20 gelagert, dessen freies Ende 21 einen Stützabschnitt 22 bildet. Dieser Stützabschnitt passt komplementär in den sich verjüngenden Schraubentrichter 38.

Die Spannzange 19 ist mit einem Zugelement 23 verbunden, das in einem Hohlschaft 27 geführt ist. Dieser Hohlschaft ist einstückig mit dem Hülsenabschnitt 20 verbunden, wobei letzterer in einem Winkel von ca. 60° gegenüber dem Hohlschaft abgewinkelt ist. Die Abwinklung erfolgt über eine Krümmung 28. Im Bereich dieser Krümmung ist das Zugelement 23 als Blattfeder 35 ausgebildet, welche auf einem im Bereich der Krümmung 28 verankerten Gleitschuh 29 aufliegt. Die Betätigung der Spannzange 19 erfolgt durch Zug in Pfeilrichtung x am Zugelement 23.

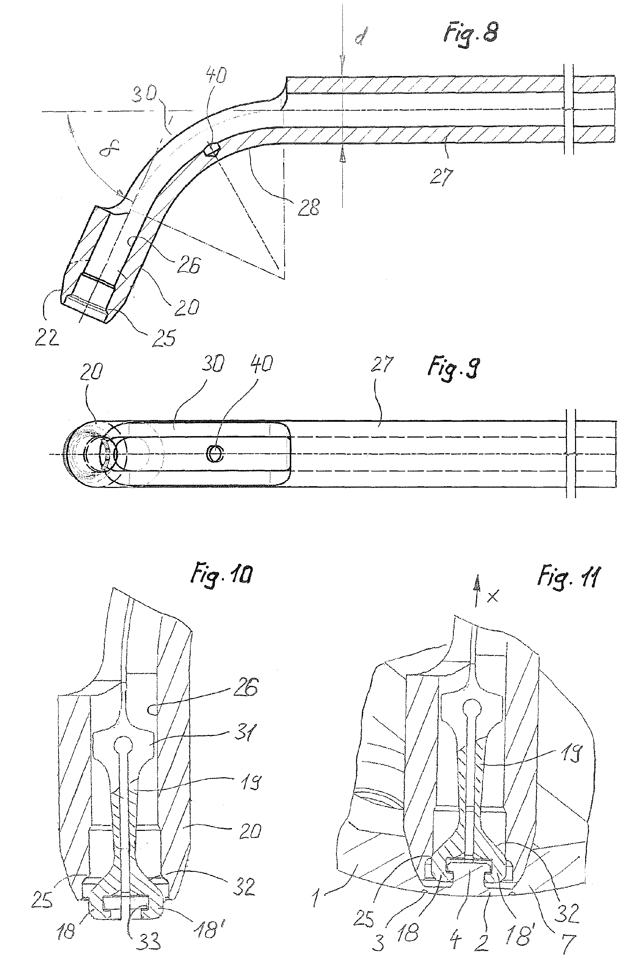

Die Figuren 8 und 9 zeigen weitere Details des Hohlschafts 27 mit dem angeformten Hülsenabschnitt 20. Dabei handelt es sich um ein Rohr mit dem Aussendurchmesser d, das mit einer Krümmung 28 derart gekrümmt wird, dass der Hülsenabschnitt 20 in einem Winkel α von ca. 60° gegenüber dem Hohlschaft ausgelenkt ist. Wie einleitend erwähnt, muss die Krümmung 28 nicht auf einem Kreisbogenabschnitt verlaufen, sondern sie kann sich gegen die geraden Abschnitte hin öffnen. Im Bereich der Krümmung ist auf der Aussenseite eine Öffnung 30 angeordnet, die sich über die gesamte Krümmung erstreckt und deren Breite etwa dem Aussendurchmesser d entspricht. An der Innenwand des gekrümmten Abschnitts ist ein Sackloch 40 angeordnet, an dem der Gleitschuh 29 gemäss Figur 7 verankert wird. Dabei könnte es sich auch um eine Durchgangsbohrung handeln. Im Bereich des Stützabschnitts 22 ist auf der Innenseite des Hülsenabschnitts 20 eine Verjüngung 25 angeordnet. Diese Verjüngung wirkt mit der Aussenseite der Klemmbacken 18, 18' zusammen, wie nachstehend noch genauer beschrieben wird.

Die Figuren 10 und 11 zeigen Details der Spannzange 19 im freigestellten und im angezogenen Zustand. Die beiden Klemmbacken 18, 18' der Spannzange 19 sind im Bereich eines Führungsabschnitts 31 einstückig miteinander verbunden, so dass sie federnd gegeneinander bewegt werden können. Der Führungsabschnitt 31 an der Spannzange wirkt mit dem Führungsabschnitt 26 auf der Innenseite des Hülsenabschnitts 20 zusammen. Auf der Aussenseite der Klemmbacken 18, 18' ist je ein Klemmrampenabschnitt 32 angeordnet, der mit der Verjüngung 25 zusammenwirken kann. Ausserdem sind die Klemmbacken mit je einer Hinterschneidung 33 versehen. Die beiden Klemmbacken 18, 18' können so angeordnet sein, dass sie zum Erfassen des Angriffmittels 4 zunächst etwas aufgespreizt werden müssen. Alternativ können sie in der Ruhelage auch bereits leicht aufgespreizt sein. Figur 11 zeigt nochmals die Situation bei einem Zug in Pfeilrichtung x auf die Spannzange 19, wobei ersichtlicherweise der Verschlussabschnitt 2 relativ zum Implantatkörper 1 ebenfalls verschoben bzw. angehoben wird. Da sich der Hülsenabschnitt 20 unmittelbar im Schraubentrichter abstützt, werden die auftretenden Biegekräfte bis zum Reissen der Sollbruchstelle 3 von dem den Schraubentrichter umgebenden Wandabschnitt 7 aufgenommen. Dadurch können ersichtlicherweise keine Kräfte auftreten, welche den Implantatkörper insgesamt beschädigen könnten.

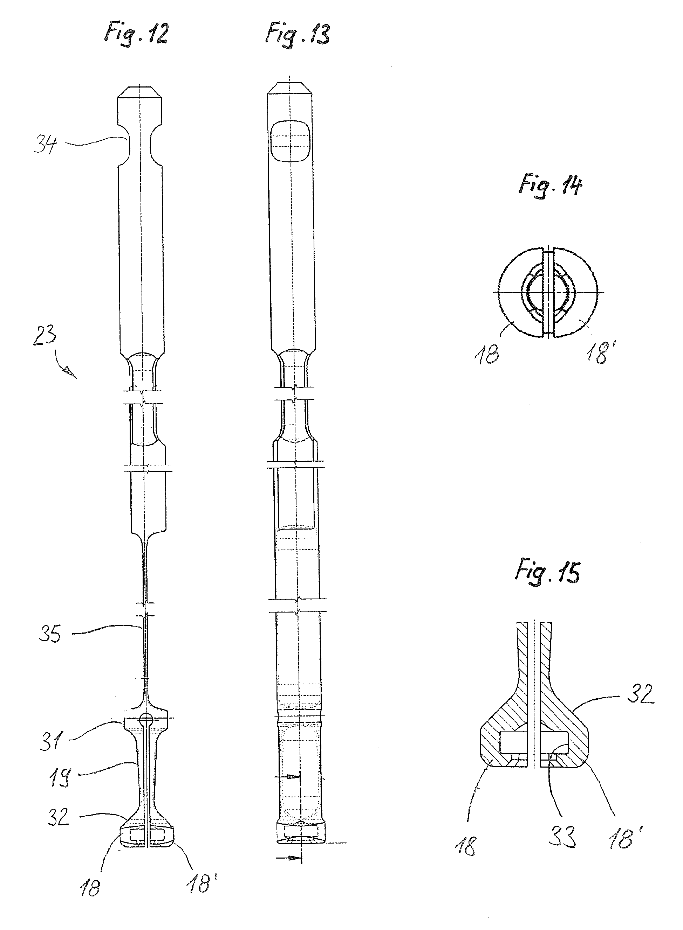

Die Figuren 12 bis 15 zeigen weitere Details des Zugelements 23. Wie dargestellt, ist die Spannzange 19 über eine Blattfeder 35 mit dem restlichen Zugelement 23 verbunden. Die Breite der Blattfeder entspricht dabei der Breite der beiden Klemmbacken 18, 18'. Der von der Spannzange 19 abgewandte Abschnitt des Zugelements 23 ist als Rohr ausgebildet, wobei an einem Einspannabschnitt 34 gegenüberliegende Aussparungen angeordnet sind. Diese dienen dazu, dass Zugelement 23 in eine Zugvorrichtung einzuspannen. Wie insbesondere aus Figur 14 ersichtlich ist, sind die beiden Klemmbacken 18, 18' im Bereich der Hinterschneidung 33 halbschalenförmig ausgebildet. Selbstverständlich kann die Konfiguration dieser Klemmbacken jeweils individuell der besonderen Form des Angriffmittels angepasst werden. Die Spannzange 19 kann ohne weiteres auch mehr als zwei Klemmbacken aufweisen.

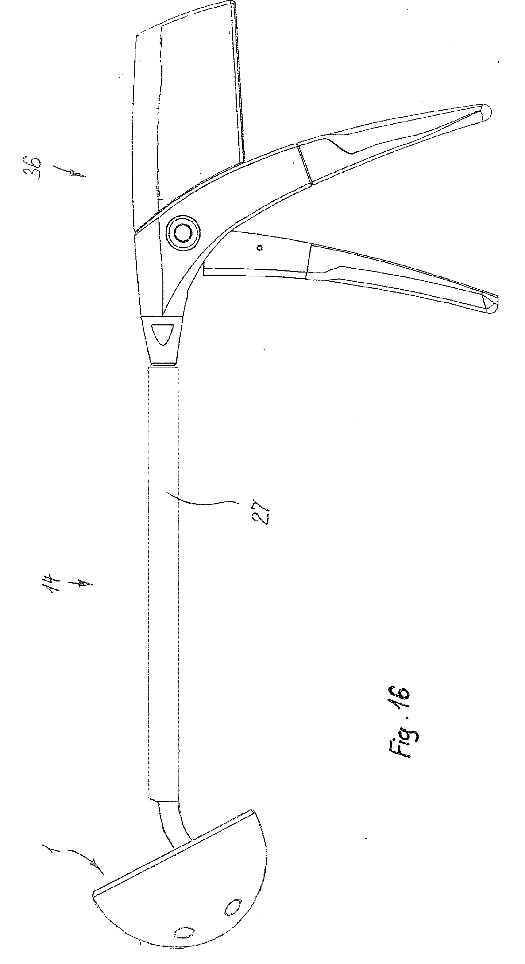

Figur 16 zeigt schematisch eine Anordnung bestehend aus einem Implantatkörper 1, einem erfindungsgemässen Instrument 14 mit manuell betätigbarer Zugvorrichtung 36. Zu diesem Zweck wird der vorstehend beschriebene Hohlschaft 27 auf geeignete Weise fest mit der Zugvorrichtung 36 verbunden, wobei diese auch noch mit dem Einspannabschnitt 34 des Zugelements 23 derart verbunden ist, dass bei einer Betätigung der Zugvorrichtung 36 eine Zugkraft ausgeübt werden kann und die Teile nicht korrodieren.

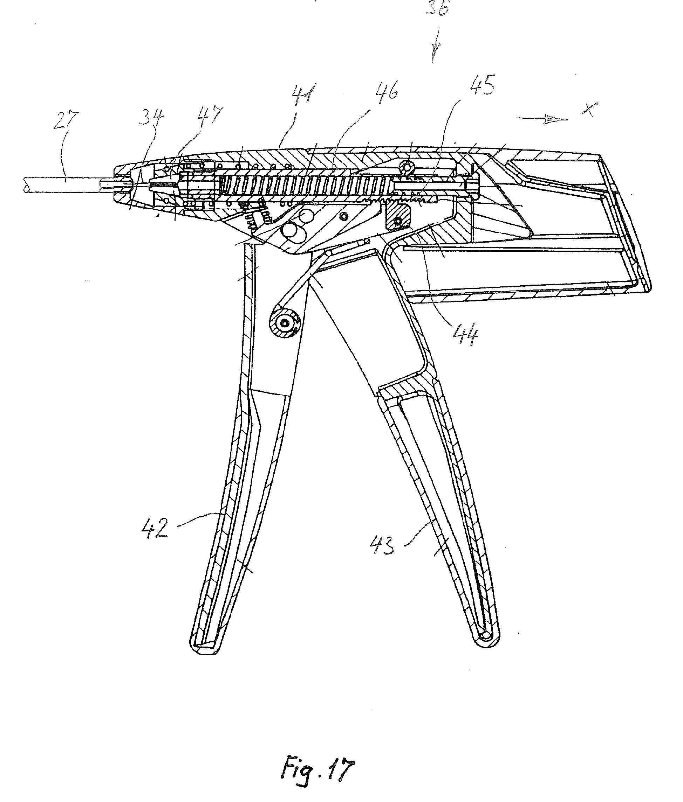

Wie bereits einleitend erwähnt, eignet sich als Zugvorrichtung besonders vorteilhaft eine Mechanik, wie sie bereits von handelsüblichen und manuell zu betätigenden Blindnietzangen bekannt ist. Eine derartige Mechanik ist beispielsweise in der EP 0 940 203 im Detail beschrieben. In Figur 17 werden daher nur die wichtigsten Elemente nochmals kurz erörtert. Demnach verfügt die Zugvorrichtung 36 über ein Gehäuse 41, in welchem ein federbelastetes Zugelement 46 gelagert ist. Der vorstehend erwähnte Hohlschaft 27 des erfindungsgemässen Instruments lässt sich fest an das Gehäuse 41 ankuppeln. Das Zugelement 46 ist mit einer Kupplung 47 versehen, welche am Einspannabschnitt 34 angreifen kann. Die Vorrichtung verfügt ausserdem über einen starr mit dem Gehäuse 41 verbundenen Griffhebel 43 und über einen schwenkbar gelagerten Hebel 42. Der Hebel 42 ist gelenkig mit einem Spannhebel 44 verbunden, der seinerseits über eine Verzahnung 45 mit dem Zugelement 46 zusammenwirken kann. Der Spannhebel 44 ist derart gelagert und vorgespannt, dass durch mehrfaches Betätigen des schwenkbaren Hebels 42 das Zugelement 46 über die Verzahnung in Pfeilrichtung x bewegt wird. Ein hier nicht näher dargestelltes Gesperre sorgt dafür, dass nach jeder Hebelbewegung das Zugelement 46 in der bereits erreichten Position verbleibt. Zum Lösen des Gesperres und damit zum Freistellen der Spannzange am Instrument muss der bewegliche Hebel 42 vom festen Hebel 43 unter Überwindung einer Arretierungskraft weggespreizt werden.

Selbstverständlich ist die Mechanik an die besonderen Bedürfnisse von chirurgischen Instrumenten anzupassen. Der bei Blindnietzangen vorhandene Vorratsbehälter zur Aufnahme der abgerissenen Nietdorne kann entfallen. Ausserdem sind die Materialien und Bauteile so zu wählen, dass die Zugvorrichtung leicht gereinigt und sterilisiert werden kann und die Teile nicht korrodieren.

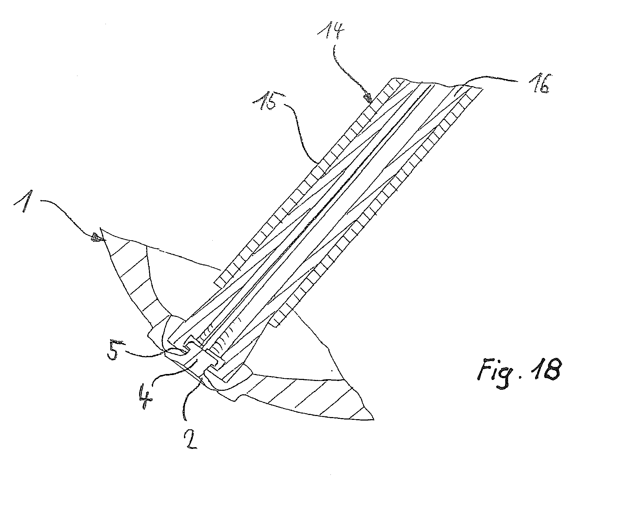

In Figur 18 ist ein alternatives Ausführungsbeispiel eines erfindungsgemässen Instruments 14 im Schnittbild gezeigt, welches mit einem Verschlussabschnitt 2 in wieder lösbarer Verbindung steht. Das Instrument 14 umfasst einen länglichen Griff 15 sowie Eingriffmittel 16. Bei der gezeigten Ausführungsform des Instruments 14 besteht das Eingriffmittel 16 aus einem länglichen Hohlkörper mit wenigstens dem gleichen Querschnitt wie die Angriffmittel. Der Griff 15 ist zumindest im Bereich von Eingriffmittel 16 ebenfalls als Hohlkörper ausgebildet, der über dem Eingriffmittel 16 verschiebbar angeordnet ist. Beim dargestellten Beispiel ist das Eingriffmittel 16 in diesem Bereich in drei Teile geteilt, welche durch sich verjüngende Schlitze voneinander getrennt sind. Durch Überschieben des Eingriffmittels 16 durch den Griff 15 verengt sich dessen Querschnitt bzw. werden die drei Teile konzentrisch gegeneinander gedrückt. Dadurch kann das Eingriffmittel 16 an das Angriffmittel eingreifen. Bevorzugt weist das Eingriffmittel 16 zusätzliche Mittel auf, die in den Hinterschnitt 5 des Angriffmittels 4 eingreifen können.

Ersichtlicherweise entspricht der Griff 15 dieses Ausführungsbeispiels dem Hülsenabschnitt 20 des oben beschriebenen Ausführungsbeispiels, und das geschlitzte Eingriffmittel 16 bildet die Spannzange. In der dargestellten Position ist der Griff 15 noch nicht am Implantatkörper 1 abgestützt. Die Relativverschiebung zwischen dem Griff 15 und dem Eingriffmittel 16 kann auf beliebige Art erfolgen.

## Patentansprüche

1. Instrument (14) zum Erfassen eines Angriffmittels (4) an einem Implantat (1), insbesondere zum Freilegen einer Öffnung (17) an einer Hüftgelenkpfanne, mit einer wenigstens zwei Klemmbacken (18) aufweisenden Spannzange (19) zum Erfassen des Angriffmittels und mit einem Hülsenabschnitt (20) zur Aufnahme der Spannzange, dessen freies Ende (21) einen an der Oberfläche des Implantats abstützbaren Stützabschnitt (22) aufweist, sowie mit Betätigungsmitteln zum Betätigen der Spannzange, **dadurch gekennzeichnet, dass** die Betätigungsmittel ein an der Spannzange (19) angreifendes Zugelement (23) aufweisen und dass die Aussenseiten der Klemmbacken derart mit der Innenseite des Hülsenabschnitts zusammenwirken, dass die Spannzange durch Zug am Zugelement schliessbar und dabei relativ zum freien Ende des Hülsenabschnitts verschiebbar ist.

2. Instrument nach Anspruch 1, **dadurch gekennzeichnet, dass** der Stützabschnitt (22) kegelstumpfförmig ausgebildet ist.

3. Instrument nach Anspruch 1 oder 2, **dadurch gekennzeichnet, dass** der Hülsenabschnitt (20) auf der Innenseite eine Verjüngung (25) aufweist, die mit Aussenseiten der Klemmbacken zusammenwirkt und dass auf der Innenseite ferner wenigstens ein vorzugsweise zylindrischer Führungsabschnitt (26) zum Führen der Spannzange angeordnet ist.

4. Instrument nach einem der Ansprüche 1 bis 3, **dadurch gekennzeichnet, dass** der Hülsenabschnitt (20) am Ende eines Hohlschafts (27) angeordnet und vorzugsweise einstückig mit diesem verbunden ist, wobei sich das Zugelement durch den Hohlschaft erstreckt.

5. Instrument nach Anspruch 4, **dadurch gekennzeichnet, dass** der Hülsenabschnitt 20 gegenüber dem Hohlschaft (27) mittels einer Krümmung (28) abgewinkelt ist.

6. Instrument nach Anspruch 5, **dadurch gekennzeichnet, dass** im Innenbereich der Krümmung (28) ein Gleitschuh (29) mit einer gekrümmten Gleitoberfläche zur Umlenkung des Zugelements angeordnet ist.

7. Instrument nach Anspruch 5 oder 6, **dadurch gekennzeichnet, dass** im Aussenbereich der Krümmung eine sich vorzugsweise über die ganze Krümmung erstreckende Öffnung (30) angeordnet ist.

8. Instrument nach einem der Ansprüche 1 bis 7, **dadurch gekennzeichnet, dass** die Klemmbacken (18) der Spannzange (19) an einem Führungsabschnitt (26) einstückig miteinander verbunden sind und dass sie auf der Aussenseite im Abstand zum Führungsabschnitt einen Klemmrampenabschnitt (32) aufweisen, der mit der Innenseite des Hülsenabschnitts zusammenwirkt.

9. Instrument nach einem der Ansprüche 1 bis 8, **dadurch gekennzeichnet, dass** die Klemmbacken (18) auf ihrer Innenseite eine Hinterschneidung (33) zum Erfassen eines Vorsprungs am Angriffmittel des Implantats aufweisen.

10. Instrument nach Anspruch 1 und Anspruch 4, **dadurch gekennzeichnet, dass** das Zugelement als Zugstange ausgebildet ist, die wenigstens abschnittsweise im Hohlschaft geführt ist.

11. Instrument nach Anspruch 10, **dadurch gekennzeichnet, dass** die Zugstange einen Einspannabschnitt (34) zum Einspannen in einer Zugvorrichtung (36) aufweist.

12. Instrument nach einem der Ansprüche 1 bis 11, **dadurch gekennzeichnet, dass** das Zugelement wenigstens abschnittsweise flexibel, vorzugsweise in der Form einer Blattfeder (35) ausgebildet ist.

13. Instrument nach einem der Ansprüche 1 bis 12, **dadurch gekennzeichnet, dass** die Spannzange einstückig mit dem Zugelement ausgebildet ist.

14. Instrument nach einem der Ansprüche 1 bis 13, **dadurch gekennzeichnet, dass** der Hülsenabschnitt und das Zugelement direkt oder indirekt sowie lösbar mit einer Zugvorrichtung (36) verbunden sind, an der eine aufgebaute Zugkraft derart aufrecht erhaltbar ist, dass sich die geschlossene Spannzange erst nach dem Lösen einer Arretierung öffnet.

15. Instrument nach Anspruch 14, **dadurch gekennzeichnet, dass** die Zugvorrichtung eine vorzugsweise handbetätigte Blindnietzange ist.

16. Verwendung einer vorzugsweise handbetätigten Blindnietzange als Zugvorrichtung zum Betätigen eines Instruments zum Erfassen eines Angriffmittels an einem Implantat, insbesondere nach einem der Ansprüche 1 bis 15.

17. Anordnung bestehend aus einem Implantat mit wenigstens einem Wandabschnitt, der wenigstens eine durch Materialumformung hergestellte Sollbruchstelle aufweist, welche zum Freilegen einer Öffnung oder Aussparung einen unter Krafteinwirkung aus dem Wandabschnitt entfernbaren Verschlussabschnitt abgrenzt, wobei der Verschlussabschnitt wenigstens ein Angriffmittel, bevorzugt einen Materialvorsprung mit Hinterschnitt zum Aufsetzen eines Instruments aufweist, sowie aus einem Instrument zum lösbaren Erfassen des Angriffmittels, insbesondere nach einem der Ansprüche 1 bis 15.

18. Instrument zum Freilegen einer Öffnung oder Aussparung an einem Implantat, insbesondere nach Anspruch 1, **dadurch gekennzeichnet, dass** das Instrument über einen länglichen Griff (15) sowie Eingriffmittel (16) verfügt, welche mit dem Verschlussabschnitt (2) lösbar verbindbar sind.

19. Instrument nach Anspruch 18, **dadurch gekennzeichnet, dass** die Eingriffmittel (16) derart ausgestaltet sind, dass sie mit auf dem Verschlussabschnitt (2) angeordneten Angriffmitteln (4) verbindbar sind.
